# EUROPEAN PATENT APPLICATION

(11) **EP 4 471 006 A1**
(43) Date of publication of application: **04.12.2024**
(21) Application number: 22924249.0
(22) Date of filing: 26.01.2022
(51) Int. Cl.: C07C 263/10, C07B 61/00, B01J 19/00, B01F 27/90, B01F 23/237, B01F 35/221

(54) **BATCH REACTOR AND METHOD FOR PREPARING ALIPHATIC ISOCYANATE**

(71) Applicant: Hanwha Solutions Corporation, Jung-gu Seoul 04541 (KR)
(72) Inventor: LEE, Hye Won, Daejeon 34128 (KR); RYU, Hyun Cheol, Daejeon 34128 (KR); HAN, Keedo, Daejeon 34128 (KR)
(74) Representative: Berggren Oy
(86) International application number: PCT/KR2022/001415
(87) International publication number: WO 2023/145991

(57) **Abstract**

A method for preparing an aliphatic isocyanate according to an exemplary embodiment of the present disclosure includes a first step of introducing an aliphatic amine and an organic solvent into a reactor, a second step of supplying gaseous hydrogen chloride into the reactor through a sparger, and a third step of stirring the aliphatic amine and the organic solvent, and the hydrogen chloride with a stirrer installed in the reactor, wherein in the third step, a stirring force of the stirrer is controlled to 500 to 4,000 W/m³.

## Description

### [Technical Field]

The present disclosure relates to a batch reaction apparatus and a method for preparing an aliphatic isocyanate.

### [Background Art]

An aliphatic isocyanate is obtained by a first-step salt formation reaction in which an amine and hydrogen chloride are reacted, and a two-step phosgenation reaction in which an amine salt produced in the salt formation reaction is reacted with phosgene.

An amine salt is formed by a reaction of hydrogen chloride supplied into an aliphatic amine and an organic solvent, and a typical batch reaction apparatus is used.

In this case, in the batch reaction apparatus, gaseous hydrogen chloride is injected into the aliphatic amine and the organic solvent introduced into a reactor, and mixing is performed with a stirrer to produce an amine salt. At this time, gas holdup of the gaseous hydrogen chloride and an amine conversion rate may be controlled by controlling a stirring force of the stirrer provided in the batch reaction apparatus.

In addition, an aliphatic isocyanate is prepared by a reaction of phosgene supplied to the amine salt produced in the first step, and a typical batch reaction apparatus is used.

In this case, in the batch reaction apparatus, gaseous phosgene is injected into the amine salt introduced into the reactor, and mixing is performed with the stirrer to prepare an aliphatic isocyanate. At this time, an amine salt conversion rate may be controlled by controlling the stirring force of the stirrer provided in the batch reaction apparatus.

### [Disclosure]

### [Technical Problem]

The present disclosure attempts to provide a batch reaction apparatus and a method for preparing an aliphatic isocyanate that may improve an amine conversion rate or an amine salt conversion rate by controlling a stirring force and may thus improve a yield of isocyanate.

### [Technical Solution]

An exemplary embodiment of the present disclosure provides a batch reaction apparatus including a reactor into which an aliphatic amine and an organic solvent are introduced, a sparger that supplies gaseous hydrogen chloride into the reactor, and a stirrer that is installed in the reactor and stirs the aliphatic amine and the organic solvent, and the hydrogen chloride, wherein a stirring force of the stirrer is 500 to 4,000 W/m³.

The stirring force of the stirrer may be 780 to 3,570 W/m³.

The stirrer may include a rotation shaft that is installed in a height direction of the reactor, and an impeller that is provided on the rotation shaft and stirs the aliphatic amine and the organic solvent, and the hydrogen chloride.

The stirrer may be provided with a radial type impeller or an axial type impeller installed at a bottom of the rotation shaft, and may be provided with one or a plurality of impellers of the same type or different types on the impeller installed at the bottom of the rotation shaft.

The stirrer may be alternately provided with the radial type impeller and the axial type impeller up to n stages as required.

The sparger may be installed at a bottom of the stirrer upward from a bottom part of the reactor, and may be provided with a plurality of injection ports to inject and supply the gaseous hydrogen chloride from the bottom of the stirrer.

The sparger may be bent at the bottom of the stirrer and may be provided with a plurality of injection ports corresponding to a rotation range of the stirrer, and the plurality of injection ports may inject the gaseous hydrogen chloride into the aliphatic amine and the organic solvent.

Another exemplary embodiment of the present disclosure provides a method for preparing an aliphatic isocyanate, the method including: a first step of introducing an aliphatic amine and an organic solvent into a reactor; a second step of supplying gaseous hydrogen chloride into the reactor through a sparger; and a third step of stirring the aliphatic amine and the organic solvent, and the hydrogen chloride with a stirrer, wherein in the third step, a stirring force of the stirrer is controlled to 500 to 4,000 W/m³.

In the third step, the stirring force of the stirrer may be controlled to 780 to 3,570 W/m³.

Yet another exemplary embodiment of the present disclosure provides a batch reaction apparatus including: a reactor that contains an amine salt in a slurry form; a sparger that supplies gaseous phosgene into the reactor; and a stirrer that is installed in the reactor and stirs the amine salt and the phosgene, wherein a stirring force of the stirrer is 500 to 10,000 W/m³.

The stirring force of the stirrer may be 1,560 to 3,170 W/m³.

The stirrer may include a rotation shaft that is installed in a height direction of the reactor, and an impeller that is provided on the rotation shaft and stirs the amine salt and the phosgene.

The stirrer may be provided with a radial type impeller or an axial type impeller installed at a bottom of the rotation shaft, and may be provided with one or a plurality of impellers of the same type or different types on the impeller installed at the bottom of the rotation shaft.

The stirrer may be alternately provided with the radial type impeller and the axial type impeller up to n stages as required.

A plurality of spargers may be provided in one direction of an axial direction of the stirrer and a circumferential direction of the reactor and may supply the gaseous phosgene.

The sparger may include a pipe that is installed on a side wall of the reactor in a direction interesting an axial direction of the stirrer, and a plurality of injection ports that are provided at an end of the pipe and supply the gaseous phosgene in a bubble state toward an impeller.

Still yet another exemplary embodiment of the present disclosure provides a method for preparing an aliphatic isocyanate, the method including: a first step of preparing a reactor containing an amine salt in a slurry form; a second step of supplying gaseous phosgene into the reactor through a sparger; and a third step of stirring the amine salt and the phosgene with a stirrer, wherein in the third step, a stirring force of the stirrer is controlled to 500 to 10,000 W/m³.

In the third step, the stirring force of the stirrer may be controlled to 1,560 to 3,170 W/m³.

### [Advantageous Effects]

In an exemplary embodiment, the stirring force of the stirrer is controlled, such that the reactivity may be controlled by controlling the gas holdup and surface area per unit volume of the gaseous hydrogen chloride, and the particle size of the amine salt particle in a slurry form may be controlled. Therefore, the amine conversion rate may be improved, and thus the yield of isocyanate, which is a final product, may be improved.

In addition, in an exemplary embodiment, the stirring force of the stirrer is controlled, such that the reactivity and the reaction rate of the phosgenation reaction may be improved. Therefore, the amine salt conversion rate may be improved, and thus the yield of isocyanate, which is a final product, may be improved.

### [Description of the Drawings]

FIG. 1 is a cross-sectional view of a batch reaction apparatus according to a first exemplary embodiment of the present disclosure.
FIG. 2 is a flowchart of a method for preparing an aliphatic isocyanate according to the first exemplary embodiment of the present disclosure.
FIG. 3 is a cross-sectional view of a batch reaction apparatus according to a second exemplary embodiment of the present disclosure.
FIG. 4 is a flowchart of a method for preparing an aliphatic isocyanate according to the second exemplary embodiment of the present disclosure.

### [Mode for Invention]

Hereinafter, exemplary embodiments of the present disclosure will be described in detail with reference to the accompanying drawings so that those skilled in the art to which the present disclosure pertains may easily practice the present disclosure. However, the present disclosure may be implemented in various different forms and is not limited to exemplary embodiments described herein. Parts irrelevant to description are omitted in the drawings in order to clearly explain the present disclosure. The same or similar components are denoted by the same reference numerals throughout the specification.

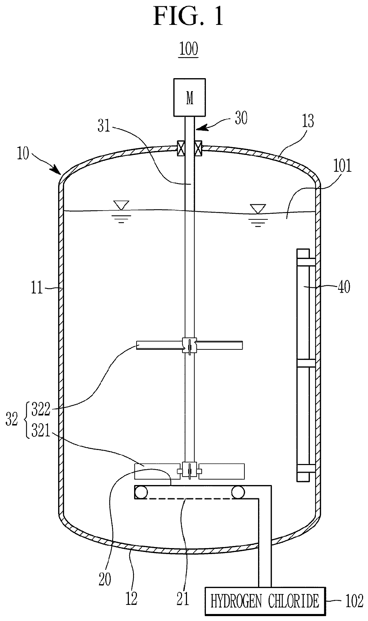

FIG. 1 is a cross-sectional view of a batch reaction apparatus according to a first exemplary embodiment of the present disclosure. Referring to FIG. 1, a batch reaction apparatus 100 according to the first exemplary embodiment includes a reactor 10 that contains an aliphatic amine and a liquid organic solvent 101, a sparger 20 that supplies gaseous hydrogen chloride 102 into the reactor 10, and a stirrer 30.

As an example, the reactor 10 includes a cylindrical side wall 11 and a bottom part 12 and a cover part 13 that form a lower side and an upper side of the side wall 11, respectively, to form a reaction space. The bottom part 12 and the cover part 13 are formed as convex curved surfaces on the lower side and the upper side, respectively, so that the aliphatic amine and the liquid organic solvent 101, and the gaseous hydrogen chloride (HCl) 102 may flow to be effectively mixed.

The sparger 20 is installed at a bottom of the stirrer 30 upward from the bottom part 12 of the reactor 10, and is provided with a plurality of injection ports 21 to inject and supply the gaseous hydrogen chloride from the bottom of the stirrer 30.

The sparger 20 is bent at the bottom of the stirrer 30 and is provided with a plurality of injection ports 21 corresponding to a rotation range of the stirrer 30. The plurality of injection ports 21 in the sparger 20 enable uniform injection of the gaseous hydrogen chloride 102 into the aliphatic amine and the liquid organic solvent 101.

The stirrer 30 includes a rotation shaft 31 that is installed in a height direction of the reactor 10, an impeller 32 that is provided on the rotation shaft 31 and stirs the aliphatic amine and the organic solvent 101, and the hydrogen chloride 102, and a motor M that drives the rotation shaft 31. The rotation shaft 31 is installed on the cover part 13 of the reactor 10 to be rotatable when the motor M is driven, and is toward the bottom part 12.

The impeller 32 includes a radial type impeller 321 and an axial type impeller 322. The radial type impeller 321 generates a flow in a transverse direction perpendicular to the rotation shaft 31 to pulverize and disperse the gaseous hydrogen chloride 102 to be supplied in the aliphatic amine and the liquid organic solvent 101. The axial type impeller 322 generates a flow in the same axial direction as the rotation shaft 31 to pulverize and disperse the gaseous hydrogen chloride 102 to be supplied in the aliphatic amine and the liquid organic solvent 101.

In the first exemplary embodiment, the radial type impeller 321 is provided as one stage on the bottom of the rotation shaft 31, and the axial type impeller 322 is provided as one stage on the rotation shaft 31 above the radial type impeller 321. That is, the radial type impeller 321 is installed in a first stage, and the axial type impeller 322 is installed in a second stage.

In the stirrer of the present disclosure, a radial type impeller or an axial type impeller may be installed at the bottom of the rotation shaft 31, and one or two or more impellers of the same or different types, that is, a plurality of impellers, may be provided thereon. In addition, in the stirrer, the radial type impeller and the axial type impeller may be alternately provided up to n stages as required. Various drawings corresponding to these cases are omitted.

The radial type impeller 321 is driven to rotate above the gaseous hydrogen chloride 102 injected through the injection ports 21 of the sparger 20, which allows the aliphatic amine and the organic solvent 101 to flow in the transverse direction. Therefore, the gaseous hydrogen chloride 102 injected through the injection ports 21 of the sparger 20 is mixed with the aliphatic amine and the organic solvent 101 while flowing in the transverse direction due to the flow of the aliphatic amine and the organic solvent 101 in the transverse direction.

The axial type impeller 322 is driven above the radial type impeller 321, which allows the aliphatic amine and the organic solvent 101 to flow in the axial direction. Therefore, the partially mixed gaseous hydrogen chloride 102 is further mixed with the aliphatic amine and the organic solvent 101 while flowing in the axial direction due to the flow of the aliphatic amine and the organic solvent 101 the axial direction.

An amine salt is produced as a salt formation reaction proceeds by mixing the hydrogen chloride 102 with the aliphatic amine and the organic solvent 101. Since the amine salt, which is a product, is in a particle form having a diameter of several to tens of micrometers (µm), as the reaction proceeds, the amine salt changes into a slurry form dispersed in the aliphatic amine and the organic solvent 101.

As described above, the combined use of the radial type impeller 321 and the axial type impeller 322 increases gas holdup when injecting the gaseous hydrogen chloride 102 into the aliphatic amine and the organic solvent 101 and mixing them. Therefore, a reaction rate in the reactor 10 may be increased. In conclusion, in the reaction of mixing the hydrogen chloride 102 with the aliphatic amine and the organic solvent 101 to produce a slurry, a uniform product may be produced in a shorter time, such that productivity may be increased and stability of product quality may be improved.

Meanwhile, the reactor 10 further includes a baffle 40 disposed in parallel with the rotation shaft 31 and installed on an inner surface of the side wall 11. For example, the baffle 40 may be formed in a plate shape having a set width and height, or may be formed in a rod shape having a set diameter and length.

When the impeller 32 is driven, the baffle 40 generates a vortex in the flowing aliphatic amine and organic solvent 101, which enables more uniform mixing of the aliphatic amine and the organic solvent 101, and the hydrogen chloride 102 throughout the entire region in the height direction of the reactor 10.

A plurality of baffles 40 are provided in a circumferential direction inside the reactor 10, and repeatedly form vortices in the circumferential direction, which enables more uniform mixing of the aliphatic amine and the organic solvent 101, and the hydrogen chloride 102 throughout the entire region in the circumferential direction.

In addition, the baffle 40 may be provided in a vortex area generated by the rotational drive of the radial type impeller 321 and the axial type impeller 322, and may directly act on mixing the aliphatic amine and the organic solvent 101, and the hydrogen chloride 102.

The use of the radial type impeller 321, the axial type impeller 322, and the baffle 40 may further increase gas holdup when injecting the gaseous hydrogen chloride 102 into the aliphatic amine and the organic solvent 101 and mixing them. Therefore, a reaction rate in the reactor 10 may be further increased.

In addition, the stirrer 20 is driven and controlled by the motor M. The gas holdup and surface area per unit volume of the gaseous hydrogen chloride 102 may be controlled by controlling a stirring force of the stirrer 30. Therefore, the reactivity of the aliphatic amine and the organic solvent 101, and hydrogen chloride 102 is controlled, and a particle size of the amine salt particle in a slurry form is controlled. Through this, an amine conversion rate is improved and a yield of aliphatic isocyanate, which is a final product, is improved.

The batch reaction apparatus 100 according to the first exemplary embodiment of the present disclosure may be used for a reaction between a gas phase and a liquid phase, and may be used in a process of preparing an aliphatic isocyanate by supplying the gaseous hydrogen chloride 102 to the aliphatic amine and the liquid organic solvent 101.

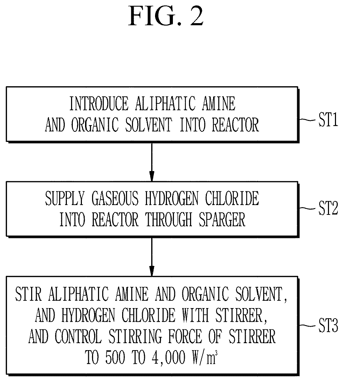

FIG. 2 is a flowchart of a method for preparing an aliphatic isocyanate according to an exemplary embodiment of the present disclosure. Referring to FIG. 2, in the method for preparing an aliphatic isocyanate, an aliphatic isocyanate is prepared using the batch reaction apparatus 100 of the first exemplary embodiment.

The method for preparing an aliphatic isocyanate includes a first step (ST1), a second step (ST2), and a third step (ST3). In the first step (ST1), an aliphatic amine and an organic solvent 101 are introduced into the reactor 10. In the second step (ST2), gaseous hydrogen chloride 102 is supplied into the reactor 10 through the sparger 20. In the third step (ST3), the aliphatic amine and the organic solvent 101, and the hydrogen chloride 102 are stirred with the stirrer 30.

In the third step (ST3), a stirring force of the stirrer 30 may be controlled by controlling the motor M. For example, in the third step (ST3), a stirring force of the stirrer 30 is controlled to 500 to 4,000 W/m³. When the stirring force is less than 500 W/m³, it is difficult to proceed the salt formation reaction due to excessively slow stirring, and when the stirring force is more than 4,000 W/m³, the effect of improving the reactivity is extremely small compared to excessively fast stirring.

In addition, the stirring force of the stirrer 30 may be controlled to 780 to 3,570 W/m³. In this range, a high salt formation reaction rate may be obtained compared to the stirring force to be applied.

Table 1 shows the stirring forces and the amine conversion rates of Example 1-1, Example 1-2, Example 1-3, and Comparative Example 1.

**[Table 1]**

| | Example 1-1 | Example 1-2 | Example 1-3 | Comparative Example 1 |
|---|---|---|---|---|
| Stirring force (W/m³) | 9730 | 3570 | 780 | <500 |
| Amine conversion rate (%) | ≥99.8 | 99.8 | 89.5 | 82.2 |

### Example 1-1

An aliphatic amine and an organic solvent 101 were placed in the batch reaction apparatus 100 of FIG. 1, and hydrogen chloride 102 was supplied through the sparger 20 to prepare an aliphatic isocyanate. In Example 1-1, the stirring force of the stirrer 30 was 9,730 W/m³.

### Example 1-2

An aliphatic amine and an organic solvent 101 were placed in the batch reaction apparatus 100 of FIG. 1, and hydrogen chloride 102 was supplied through the sparger 20 to prepare an aliphatic isocyanate. In Example 1-2, the stirring force of the stirrer 30 was 3,570 W/m³.

### Example 1-3

An aliphatic amine and an organic solvent 101 were placed in the batch reaction apparatus 100 of FIG. 1, and hydrogen chloride 102 was supplied through the sparger 20 to prepare an aliphatic isocyanate. In Example 1-3, the stirring force of the stirrer 30 was 780 W/m³.

### Comparative Example1

An aliphatic amine and an organic solvent 101 were placed in the batch reaction apparatus 100 of FIG. 1, and hydrogen chloride 102 was supplied through the sparger 20 to prepare an aliphatic isocyanate. In Comparative Example 1, the stirring force of the stirrer 30 was less than 500 W/m³.

### Experimental Example 1

In Example 1-1, Example 1-2, Example 1-3, and Comparative Example 1, the amine conversion rate (%) according to the stirring force was measured. In Example 1-1, when the stirring force of the stirrer 30 was 9,730 W/m³, the amine conversion rate (%) was 99.8% or more, in Example 1-2, when the stirring force of the stirrer 30 was 3,570 W/m³, the amine conversion rate (%) was 99.8%, in Example 1-3, when the stirring force of the stirrer 30 was 780 W/m³, the amine conversion rate (%) was 89.5%, and in Comparative Example 1, when the stirring force of the stirrer 30 was less than 500 W/m³, the amine conversion rate (%) was 82.2%. Therefore, Examples 1-1, 1-2, and 1-3 may improve the amine conversion rate and improve the yield of aliphatic isocyanate, which is a final product, compared to Comparative Example 1.

Hereinafter, the second exemplary embodiment of the present disclosure will be described.

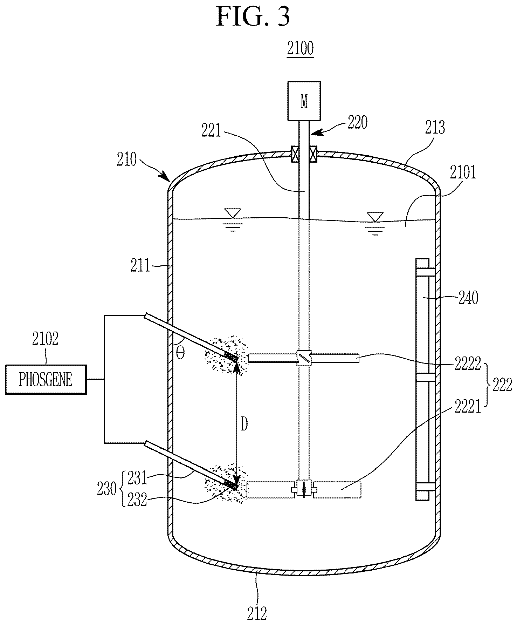

FIG. 3 is a cross-sectional view of a batch reaction apparatus according to a second exemplary embodiment of the present disclosure. Referring to FIG. 3, a batch reaction apparatus 2100 according to the second exemplary embodiment includes a reactor 210 that contains an amine salt 2101 in a slurry form produced in a salt formation reaction, a sparger 230 that supplies gaseous phosgene 2102 into the reactor 210, and a stirrer 220.

As an example, the reactor 210 includes a cylindrical side wall 211 and a bottom part 212 and a cover part 213 that form a lower side and an upper side of the side wall 211, respectively, to form a reaction space. The bottom part 212 and the cover part 213 are formed as convex curved surfaces on the lower side and the upper side, respectively, so that the amine salt 2101 in a slurry form and the gaseous phosgene 2102 may flow to be effectively mixed.

The stirrer 220 includes a rotation shaft 221 that is installed in a height direction of the reactor 210, an impeller 222 that is provided on the rotation shaft 221 and stirs the amine salt 2101 and the phosgene 2102, and a motor M that drives the rotation shaft 221. The rotation shaft 221 is installed on the cover part 213 of the reactor 210 to be rotatable when the motor M is driven, and is toward the bottom part 212.

The impeller 222 includes a radial type impeller 2221 and an axial type impeller 2222. The radial type impeller 2221 generates a flow in a transverse direction perpendicular to the rotation shaft 221 to pulverize and disperse the gaseous phosgene 2102 to be supplied in the amine salt 2101. The axial type impeller 2222 generates a flow in the same axial direction as the rotation shaft 221 to pulverize and disperse the gaseous phosgene 2102 to be supplied in the amine salt 2101.

In the second exemplary embodiment, the radial type impeller 2221 is provided as one stage on the bottom of the rotation shaft 221, and the axial type impeller 2222 is provided as one stage on the rotation shaft 221 above the radial type impeller 2221. That is, the radial type impeller 2221 is installed in a first stage, and the axial type impeller 2222 is installed in a second stage.

In the stirrer of the present disclosure, a radial type impeller or an axial type impeller may be installed at the bottom of the rotation shaft 221, and one or two or more impellers of the same or different types, that is, a plurality of impellers, may be provided thereon. In addition, in the stirrer, the radial type impeller and the axial type impeller may be alternately provided up to n stages as required. Various drawings corresponding to these cases are omitted.

A plurality of spargers 230 are provided in one direction of an axial direction of the stirrer 220 and a circumferential direction of the reactor 210 and are formed and installed to supply the gaseous phosgene 2102. According to the second exemplary embodiment, the plurality of spargers 230 are installed on the side wall 211 of the reactor 210 at a set interval D in the axial direction of the stirrer 220.

The sparger 230 includes a pipe 231 and a bubble forming part 232. The pipe 231 is installed on the side wall 211 of the reactor 210 with its longitudinal direction intersecting the axial direction of the stirrer 220. As an example, the pipe 231 is installed on the side wall 211 at a set angle θ.

The bubble forming part 232 is provided with a plurality of injection ports at an end of the pipe 231 and supplies the gaseous phosgene 2102 in a bubble state toward the impeller 222. The plurality of spargers 230 are provided in the reactor 210 in the axial direction, which enables uniform distribution of the gaseous phosgene 2102 in the axial direction.

When the plurality of impellers 222 are provided, the spargers 230 may be installed corresponding to the impellers 222. Therefore, each impeller 222 uniformly distributes the gaseous phosgene 2102 supplied from each sparger 230 within the interval D, such that the gaseous phosgene 2102 may be distributed more uniformly in the entire axial direction of the reactor 210.

Meanwhile, the radial type impeller 2221 is driven to rotate around the gaseous phosgene 2102 supplied from the bubble forming part 232 of the sparger 230, which allows the amine salt 2101 to flow in the transverse direction. Therefore, the gaseous phosgene 2102 supplied through the bubble forming part 232 of the sparger 230 is mixed with the amine salt 2101 while flowing in the transverse direction due to the flow of the amine salt 2101 in the transverse direction.

The axial type impeller 2222 is driven above the radial type impeller 2221, which allows the amine salt 2101 to flow in the axial direction. Therefore, the partially mixed gaseous phosgene 2102 and gaseous phosgene 2102 are further mixed with the amine salt 2101 while flowing in the axial direction due to the flow of the amine salt 2101 in the axial direction.

As described above, the plurality of spargers 230 arranged in the axial direction uniformly distribute and supply the gaseous phosgene 2102 to the amine salt 2101, which increases gas holdup within a predetermined time. In conclusion, the distribution performance of phosgene to the amine salt 2101 in a slurry state may be improved in the phosgenation reaction that occurs by supplying the phosgene 2102 to the amine salt 2101. Therefore, a reaction rate in the reactor 210 may be increased.

Meanwhile, the reactor 210 further includes a baffle 240 disposed in parallel with the rotation shaft 221 and installed on an inner surface of the side wall 211. For example, the baffle 240 may be formed in a plate shape having a set width and height, or may be formed in a rod shape having a set diameter and length.

When the impeller 222 is driven, the baffle 240 generates a vortex in the flowing amine salt 2101, which enables more uniform mixing of the amine salt 2101 and the phosgene 2102 throughout the entire region in the height direction of the reactor 210.

A plurality of baffles 240 are provided in the circumferential direction inside the reactor 210, and repeatedly form vortices in the circumferential direction, which enables more uniform mixing of the amine salt 2101 and the phosgene 2102 throughout the entire region in the circumferential direction.

In addition, the baffle 240 may be provided in a vortex area generated by the rotational drive of the radial type impeller 2221 and the axial type impeller 2222, and may directly act on mixing the amine salt 2101 and the phosgene 2102.

In addition to the sparger 230, the use of the radial type impeller 2221, the axial type impeller 2222, and the baffle 240 may further increase gas holdup when injecting the gaseous phosgene 2102 into the amine salt 2101 and mixing them. Therefore, a reaction rate in the reactor 210 may be further increased.

In addition, the stirrer 220 is driven and controlled by the motor M. The stirrer 220 may improve the reactivity and the reaction rate of the phosgenation reaction by controlling the stirring force. Therefore, an amine salt conversion rate is improved and a yield of aliphatic isocyanate, which is a final product, is improved.

The batch reaction apparatus 2100 according to the second exemplary embodiment of the present disclosure may be used for a reaction between a gas phase and a slurry phase, and may be used in a process of preparing an aliphatic isocyanate by supplying the gaseous phosgene 2102 to the amine salt 2101.

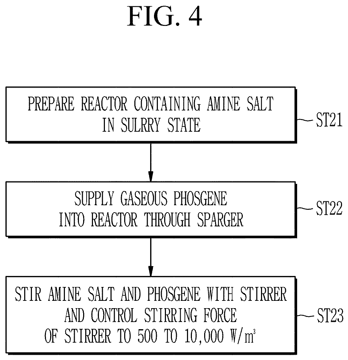

FIG. 4 is a flowchart of a method for preparing an aliphatic isocyanate according to the second exemplary embodiment of the present disclosure. Referring to FIG. 4, in the method for preparing an aliphatic isocyanate, an aliphatic isocyanate is prepared using the batch reaction apparatus 2100 of the second exemplary embodiment.

The method for preparing an aliphatic isocyanate includes a first step (ST21), a second step (ST22), and a third step (ST23). In the first step (ST21), the reactor 210 containing an amine salt in a slurry state is prepared. In the second step (ST22), gaseous phosgene 2102 is supplied into the reactor 210 through the sparger 230. In the third step (ST23), an amine salt 2101 in a slurry state and the phosgene 2102 are stirred with the stirrer 220.

In the third step (ST23), a stirring force of the stirrer 220 may be controlled by controlling the motor M. For example, in the third step (ST23), a stirring force of the stirrer 220 is controlled to 500 to 10,000 W/m³. When the stirring force is less than 500 W/m³, it is difficult to proceed the phosgenation reaction due to excessively slow stirring, and when the stirring force is more than 10,000 W/m³, the effect of improving the reactivity is extremely small compared to excessively fast stirring.

In addition, the stirring force of the stirrer 220 may be controlled to 1,560 to 31,700 W/m³. In this range, a high phosgenation reaction rate may be obtained compared to the stirring force to be applied.

Table 2 shows the stirring forces and the amine salt conversion rates of Example 2-1, Example 2-2, Example 2-3, and Comparative Example 2.

**[Table 2]**

| | Example 2-1 | Example 2-2 | Example 2-3 | Comparative Example 2 |
|---|---|---|---|---|
| Stirring force (W/m³) | 14200 | 3170 | 1560 | <500 |
| Amine salt conversion rate (%) | 99.3 | 99.2 | 97.8 | 81.12 |

### Example 2-1

An amine salt 2101 was placed in the batch reaction apparatus 2100 of FIG. 3, and gaseous phosgene 2102 was supplied to the amine salt 2101 through the sparger 230 to prepare an aliphatic isocyanate. In Example 2-1, the pipe type sparger 230 was used in two stages in the axial direction. The stirring force of the stirrer 220 was 14,200 W/m³.

### Example 2-2

An amine salt 2101 was placed in the batch reaction apparatus 2100 of FIG. 3, and gaseous phosgene 2102 was supplied to the amine salt 2101 through the sparger 230 to prepare an aliphatic isocyanate. In Example 2-2, the pipe type sparger 230 was used in two stages in the axial direction. The stirring force of the stirrer 220 was 3,170 W/m³.

### Example 2-3

An amine salt 2101 was placed in the batch reaction apparatus 2100 of FIG. 3, and gaseous phosgene 2102 was supplied to the amine salt 2101 through the sparger 230 to prepare an aliphatic isocyanate. In Example 2-3, the pipe type sparger 230 was used in two stages in the axial direction. The stirring force of the stirrer 220 was 1,560 W/m³.

### Experimental Example 2

In Example 2-1, Example 2-2, Example 2-3, and Comparative Example 2, the amine salt conversion rate (%) according to the stirring force was measured. In Example 2-1, when the stirring force of the stirrer 220 was 14,200 W/m³, the amine salt conversion rate (%) was 99.3%, in Example 2-2, when the stirring force of the stirrer 220 was 3,170 W/m³, the amine salt conversion rate (%) was 99.2%, in Example 2-3, when the stirring force of the stirrer 220 was 1,560 W/m³, the amine salt conversion rate (%) was 97.8%, and in Comparative Example 2, when the stirring force of the stirrer 220 was less than 500 W/m³, the amine salt conversion rate (%) was 81.12%.

Therefore, compared to Comparative Example 2, Examples 2-1, 2-2, and 2-3 may improve the reactivity and the reaction rate of the phosgenation reaction and may improve the amine salt conversion rate, and thus, the yield of isocyanate, which is a final product, may be improved.

Although preferred exemplary embodiments of the present disclosure have been described, the present disclosure is not limited to the exemplary embodiments, but may be implemented in various modifications within the scope of the claims, the description of the disclosure, and the accompanying drawings. These modifications also fall within the scope of the present disclosure.

### (Description of symbols)

| | | | |
|---|---|---|---|
| 10: | Reactor | 11: | Side wall |
| 12: | Bottom part | 13: | Cover part |
| 20: | Sparger | 21: | Injection port |
| 30: | Stirrer | 31: | Rotation shaft |
| 32: | Impeller | 40: | Baffle |
| 100: | Rotary reaction apparatus | 101: | Organic solvent |
| 102: | Hydrogen chloride | 321: | Radial type impeller |
| 322: | Axial type impeller | M: | Motor |
| 210: | Reactor | 211: | Side wall |
| 212: | Bottom part | 213: | Cover part |
| 220: | Stirrer | 221: | Rotation shaft |
| 222: | Impeller | 230: | Sparger |
| 231 | Pipe | 232: | Bubble forming part |
| 240: | Baffle | 2100: | Rotary reaction apparatus |
| 2101: | Amine salt | 2102: | Phosgene |
| 2221: | Radial type impeller | 2222: | Axial type impeller |

## Claims

1. A batch reaction apparatus comprising:
a reactor into which an aliphatic amine and an organic solvent are introduced;
a sparger that supplies gaseous hydrogen chloride into the reactor; and
a stirrer that is installed in the reactor and stirs the aliphatic amine and the organic solvent, and the hydrogen chloride,
wherein a stirring force of the stirrer is 500 to 4,000 W/m³.

2. The batch reaction apparatus of claim 1, wherein:
the stirring force of the stirrer is 780 to 3,570 W/m³.

3. The batch reaction apparatus of claim 1, wherein:
the stirrer includes
a rotation shaft that is installed in a height direction of the reactor, and
an impeller that is provided on the rotation shaft and stirs the aliphatic amine and the organic solvent, and the hydrogen chloride.

4. The batch reaction apparatus of claim 3, wherein:
the stirrer is provided with a radial type impeller or an axial type impeller installed at a bottom of the rotation shaft, and
is provided with one or a plurality of impellers of the same type or different types on the impeller installed at the bottom of the rotation shaft.

5. The batch reaction apparatus of claim 4, wherein:
the stirrer is alternately provided with the radial type impeller and the axial type impeller up to n stages as required.

6. The batch reaction apparatus of claim 1, wherein:
the sparger is installed at a bottom of the stirrer upward from a bottom part of the reactor, and is provided with a plurality of injection ports to inject and supply the gaseous hydrogen chloride from the bottom of the stirrer.

7. The batch reaction apparatus of claim 6, wherein:
the sparger is bent at the bottom of the stirrer and is provided with a plurality of injection ports corresponding to a rotation range of the stirrer, and
the plurality of injection ports inject the gaseous hydrogen chloride into the aliphatic amine and the organic solvent.

8. A method for preparing an aliphatic isocyanate, the method comprising:
a first step of introducing an aliphatic amine and an organic solvent into a reactor;
a second step of supplying gaseous hydrogen chloride into the reactor through a sparger; and
a third step of stirring the aliphatic amine and the organic solvent, and the hydrogen chloride with a stirrer,
wherein in the third step,
a stirring force of the stirrer is controlled to 500 to 4,000 W/m³.

9. The method of claim 8, wherein:
in the third step,
the stirring force of the stirrer is controlled to 780 to 3,570 W/m³.

10. A batch reaction apparatus comprising:
a reactor that contains an amine salt in a slurry form;
a sparger that supplies gaseous phosgene into the reactor; and
a stirrer that is installed in the reactor and stirs the amine salt and the phosgene,
wherein a stirring force of the stirrer is 500 to 10,000 W/m³.

11. The batch reaction apparatus of claim 10, wherein:
the stirring force of the stirrer is 1,560 to 3,170 W/m³.

12. The batch reaction apparatus of claim 10, wherein:
the stirrer includes
a rotation shaft that is installed in a height direction of the reactor, and
an impeller that is provided on the rotation shaft and stirs the amine salt and the phosgene.

13. The batch reaction apparatus of claim 12, wherein:
the stirrer is provided with a radial type impeller or an axial type impeller installed at a bottom of the rotation shaft, and
is provided with one or a plurality of impellers of the same type or different types on the impeller installed at the bottom of the rotation shaft.

14. The batch reaction apparatus of claim 13, wherein:
the stirrer is alternately provided with the radial type impeller and the axial type impeller up to n stages as required.

15. The batch reaction apparatus of claim 10, wherein:
a plurality of spargers are provided in one direction of an axial direction of the stirrer and a circumferential direction of the reactor and supply the gaseous phosgene.

16. The batch reaction apparatus of claim 10, wherein:
the sparger includes
a pipe that is installed on a side wall of the reactor in a direction interesting an axial direction of the stirrer, and
a plurality of injection ports that are provided at an end of the pipe and supply the gaseous phosgene in a bubble state toward an impeller.

17. A method for preparing an aliphatic isocyanate, the method comprising:
a first step of preparing a reactor containing an amine salt in a slurry form;
a second step of supplying gaseous phosgene into the reactor through a sparger; and
a third step of stirring the amine salt and the phosgene with a stirrer,
wherein in the third step,
a stirring force of the stirrer is controlled to 500 to 10,000 W/m³.

18. The method of claim 17, wherein:
in the third step,
the stirring force of the stirrer is controlled to 1,560 to 3,170 W/m³.
